# EUROPEAN PATENT SPECIFICATION

(11) **EP 1 361 885 B1**
(45) Date of publication and mention of the grant of the patent: **16.04.2008**
(21) Application number: 02718150.2
(22) Date of filing: 20.02.2002
(51) Int. Cl.: A61K 38/06, A61P 27/16, A61P 13/00

(54) **USE OF GAMMA-GT INHIBITORS FOR THE TREATMENT OF CHRONIC DEGENERATIVE DISEASES**
VERWENDUNG VON GAMMA-GT INHIBITOREN ZUR BEHANDLUNG VON CHRONISCHEN DEGENERATIVERKRANKUNGEN
UTILISATION D'INHIBITEURS GAMMA-GT POUR TRAITER DES MALADIES DEGENERATIVES CHRONIQUES

(30) Priority: 20.02.2001 EP 01104063
(43) Date of publication of application: 19.11.2003
(73) Proprietor: GTX Pharmaceuticals GmbH, 53111 Bonn (DE); Weiher, Hans, 53111 Bonn (DE)
(72) Inventor: WEIHER, Hans, 53111 Bonn (DE); SIES, Helmut, 40667 Meerbusch (DE); WAGNER, Günter, 40591 Düsseldorf (DE)
(74) Representative: Vossius & Partner
(86) International application number: PCT/EP2002/001799
(87) International publication number: WO 2002/066047

(56) References cited:
- US-A- 4 758 551
- TOWNSEND DANYELLE M ET AL: "In vivo metabolism of cisplatin to a nephrotoxin by gamma-glutamyl transpeptidase and beta-lyase." PROCEEDINGS OF THE AMERICAN ASSOCIATION FOR CANCER RESEARCH ANNUAL, no. 41, March 2000 (2000-03), page 266 XP001084090 91st Annual Meeting of the American Association for Cancer Research.;San Francisco, California, USA; April 01-05, 2000, March, 2000 ISSN: 0197-016X
- HANIGAN M H ET AL: "Human germ cell tumours: Expression of gamma-glutamyl transpeptidase and sensitivity to cisplatin." BRITISH JOURNAL OF CANCER, vol. 81, no. 1, 1999, pages 75-79, XP001083693 ISSN: 0007-0920
- DATABASE BIOSIS [Online] BIOSCIENCES INFORMATION SERVICE, PHILADELPHIA, PA, US; August 1998 (1998-08) HANIGAN MARIE H ET AL: "Expression of gamma-glutamyl transpeptidase in stage III and IV ovarian surface epithelial carcinomas does not alter response to primary cisplatin-based chemotherapy." Database accession no. PREV199800451043 XP002203802 & AMERICAN JOURNAL OF OBSTETRICS AND GYNECOLOGY, vol. 179, no. 2, August 1998 (1998-08), pages 363-367, ISSN: 0002-9378
- EVANS P., HALLIWELL B: "Free radicals and hearing: cause, consequence and criteria" ANNALS OF THE NEW YOORK ACADEMY OF SCIENCES, [Online] vol. 884, no. 1, 1999, pages 19-40, XP002203800 Retrieved from the Internet: <URL:www.annalsnyas.org> [retrieved on 2002-06-27]
- LOPEZ-GONZALEZ MIGUEL A ET AL: "Ototoxicity caused by cisplatin is ameliorated by melatonin and other antioxidants." JOURNAL OF PINEAL RESEARCH., vol. 28, no. 2, March 2000 (2000-03), pages 73-80, XP002203801 ISSN: 0742-3098
- DATABASE BIOSIS [Online] BIOSCIENCES INFORMATION SERVICE, PHILADELPHIA, PA, US; 1996 NISHIDA ISAO ET AL: "Attenuation of aminoglycoside ototoxicity by glutathione." Database accession no. PREV199699048528 XP002203803 & ORL (BASEL), vol. 58, no. 2, 1996, pages 68-73, ISSN: 0301-1569
- KIL J ET AL: "Localization of gamma-glutamyl transpeptidase in the chick inner ear sensory epithelia." SOCIETY FOR NEUROSCIENCE ABSTRACTS, vol. 22, no. 1-3, 1996, page 1621 XP001083810 26th Annual Meeting of the Society for Neuroscience;Washington, D.C., USA; November 16-21, 1996 ISSN: 0190-5295

## Description

The present invention relates to the use of γ-GT inhibitors for the preparation of pharmaceutical compositions for the treatment of degenerative diseases. In particular it pertains to the treatment of chronic renal or inner ear conditions or injuries which are reactive oxygen species (ROS) induced.

Degenerative diseases are considered to be diseases which are linked to chronic disorders and/or chronic physiological damages in the human or animal body.
Besides degenerative diseases of, inter alia, the centrol nervous system, chronic disorders of the kidneys or the liver may lead to degeneration of the corresponding tissues. For example renal diseases, or inner ear degenerative diseases are frequent pathological conditions for which hardly any treatment is available.

In particular, glomerulosclerosis and other renal diseases are a frequent complication of many chronic conditions including diabetes (Ha and Kim, Diabetes Res. Clin. Pract. 45 (1999), 147-151). Thereby an excess of reactive oxygen species (ROS) is thought to play a crucial role (Haugen and Nath, Blood Purif. 17 (1999), 58-65). Animal models have been developed to study the pathomechenisms involved. In a genetic mouse model of otorenal disease (Meyer zum Gottesberge, European Archiv Otorhinolaryngology 253 (1996), 470-474; Weiher, Cell 62 (1990), 425-434) it has been shown that interventional therapy using radical scavengers is effective (Binder, Am. J. Pathol. 154 (1999), 1067-1075). However, antioxidant therapy in humans would be rather difficult to carry out with the desired specificity, considering, for instance, the particular oxygen species to be scavenged as well as its site of action. Therefore, it would be advantageous to inhibit specifically the enzymatic activity that generates the particular ROS responsible for the tissue damage.

If the prior art has proposed a role of ROS in glomerulosclerosis nothing was known about the actual source of the damaging enities in said disease. Recently, reaction conditions have been defined in vitro, in which ROS are formed as a consequence of the action of the enzyme γ-GT ((Drozdz *et al.,* 1998)).

Although an important role of ROS in the pathological-mechanism of glomerulosclerosis has been recognized in the prior art, it was not known which of the many possible mechanisms to generate said oxygen species is responsible.

Therefore, the technical problem of the present invention was to provide for means and methods which may be used in the treatment and/or alleviation of degenerative, chronic diseases. The solution to said technical problem is achieved by providing the embodiments characterized in the claims.

Thus, the present invention relates to the use of γ-GT inhibitors for the preparation of a pharmaceutical composition for the treatment of a chronic degenerative disease, wherein said chronic degenerative disease is a chronic renal disease or a chronic inner ear degenerative condition or injury.

In this invention it was surprisingly found that overexpression of the enzyme γGT is a source of damaging ROS in the kidney and other cells, in particular of cells of the inner ear. Thus, the inhibition of γGT (systemic or local) should lead to means and methods for successfully and effectively preventing the progress of the chonic tissue damage imposed by elevated ROS levels in kidney and inner ear.

Drozdz (Free Radical Biology and Medicine 25 (1998), 786-792) has speculated based on an in vitro experiment, that γ-GT may be involved in the generation of ROS. Yet, these investigations provoked an ROS generation in vitro by applying a glutathione/transferrin system to cultured V79 cells. These authors showed a rather fast ROS generation by γ-GT when glutathione and transferrin was present. This in vitro generation of ROS reached a maximal rate in the first six minutes. However, implications for longer and/or constant damage by ROS in an in vivo situation could not be drawn by these published data.

Furthermore, in this paper it was not addressed whether this mechanism had any relevance on the organismal level in vivo. There were no implications for a role of this in persistent damage by ROS leading to chronic tissue lesions.
Yet, in this invention it could be surprisingly shown (see appended examples) that excess γ-GT activity and increased extracellular ROS can be found in vivo. In addition, and in contrast to Drodz et al. (loc. cit., 1998), in this invention it could be demonstrated, that in a model of chronic disease the increased extracellular ROS, identified as causal for the disease, was accompanied by excess γGT activity.
Furthermore, it was found that the membrane localised γ-GT enzyme, which commonly is described as a oxygen protective activity (by enhancing intracellular Cys-Gly levels, and therefore allowing for intracellular accumulation of glutathione), actually produces extracellular Cys-Gly concentrations involved in ROS formation and that excess γ-GT activity results in increased extracellular ROS in in vivo situations. It can therefore be reasoned, that the membrane localised γ-GT causes ROS-mediated chronic damages.
Therefore, the present invention provides for the above mentioned use of γ-GT inhibitors in degenerative chronic diseases.
γ-GT inhibitors and their medical use are known in the art. Inter alia, US 4,758,551 describes the use of such inhibitors for γ-GT in the protection of kidneys from metal or toxin poisoning. However, in US 4,758,551 it was speculated that γ-GT activity allows for the uptake and metabolism of these toxic substances. A link to degenerative, chronic diseases was neither shown nor speculated.

Townsend (2000) Proceeding of the American Association for Cancer Research, 41, page 266 teaches that a pretreatment with γ-GT inhibitors or with an inhibitor of β-lyase protects mice from nephrotoxicity induced by cisplatin. Also Hanigan (1996) Am. J. Obest. and Gynecol. 275, 270-275 and Hanigen (1998) Am. J. of Obest and Gynecol. 179, 363-367 relate to the pre-treatment of mice with γ-GT inhibitors in order to circumvent acute nephrotoxicity (Hanigan (1996)) or to the correlation between high levels of γ-GT in tumor and acute ototoxicity in patients treated with cisplatin (Hanigan (1998)). Evans (1999) Anal. of New York Academy of Sciences, 884, pp. 19-40 describes the damaging effect of the administration of the antibiotic aminoglycoside and of cisplatin and teaches that these substances lead to an ototoxicity. Lopez-Gonzalez (2000) J. Pineal Research 28, 73-80 administered α-tocopherol acid succinate, asorbic acid, glutathione and N-acetylcysteine to rats suffering from an acute ototoxicity related to cisplatin administration. Isao (1996) Database Biosys (Online) teaches that amino glycoside ototoxicity can be attenuated by the administration of glutathione. The glutathione was administered prior to administration of amikacin. This pre-treatment reduced the damage of outer hair cells. Kil (1996) Society of Neuroscience Vol. 22, pp. 1621 (abstract) compares the effect of amino glycosides and of cisplatin in ototoxicity and nephrotoxicity. It is taught that γ-GT levels may be inhibited by acivicin and that this inhibition prevents destruction of renal proximal tubule cells induced by the intoxination with cisplatin.

Degenerative diseases according to the invention are chronic renal diseases or inner ear degenerative diseases or injuries, whereby these diseases are chronic disorders and/or ROS-induced.

Particularly preferred examples of chronic renal diseases are focal and/or segmental glomerulosclerosis, minimal change nephrosis, inflammatory and/or autoimmune glomerulopathies. Yet, it is also envisaged that said chronic renal disease is diabetic nephropathy.

In the context of the invention, particularly preferred examples of inner ear injuries are sensineural deafness induced by age, physiological status and/or metabolical status. A preferred example for a chronic inner ear degenerative condition according to the invention is otosclerosis.

Particularly preferred γ-GT-inhibitors in context of this invention and which may be employed in the above described use are AT 125 (Acivicin) or its derivatives. In a preferred embodiment, peptide inhibitors of the CysGlyXtype may be employed, wherein X may either stand for any of the naturally occurring aminoacids or a modified aminoacid, may be used according to the invention. Further, peptide inhibitors are inhibitors γ-GluXYtype, wherein X and Y may either stand for any of the naturally occurring aminoacids or a modified aminoacid, may be used according to the invention. Furthermore, peptidomimetic glutathione analogues (Burg, Bioorg Med Chem 10 (2002),195-205), compounds or derivatives of the type L-2-amino-4-boronobutanoic acid (ABBA) (London and Gabel, Arch Biochem Biophys 385 (2001), 250-258), and anilides, such as γ-glutamyl-7-amido-4-methylcoumarin (γ-Glu-AMC) (Stein, Biochemistry 40 (2001), 5804-5811) may be used in accordance with this invention.

The pharmaceutical compositions according to the invention can be administered by any appropriate route such as oral, buccal, sublingual, parenteral, topical or by inhalation and can be formulated according to methods well known in the art. Accordingly, the present invention also provides for a method of treatment, prevention and/or amelioration of a degenerative disease, in particular of chronic renal diseases or inner ear degenerative conditions or inner ear injuries comprising the step of administering to a patient in need thereof a pharmaceutical composition as defined herein. Preferably said patient is a human patient.

Typically an effective amount of the effective compound will be admixed with a pharmaceutically acceptable carrier, optionally using further excipients known to the person skilled in the art if appropriate.

It will be appreciated by the person skilled in the art that the route of application and the daily dosis is dependent on individual parameters such as severity of the condition/disease treated, concommitant diseases and medication, age and body weight of the patient.

The Figures show:

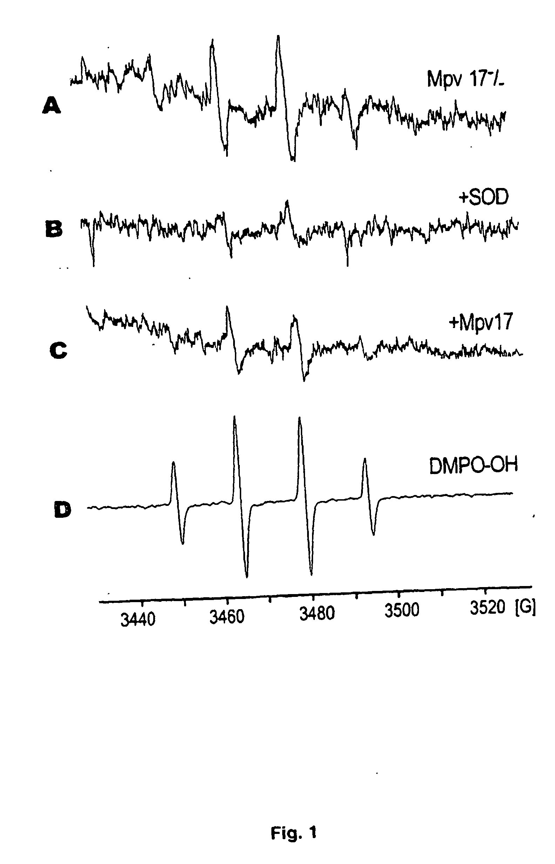

- **Figure 1**:: ESR spectra from DMPO-OH adducts produced in fibroblasts. A, B: Mpv17-/- (LUSVX) cells were reacted with DMPO for 60 min. B: Reaction as in A in the presence of 30 units/ml SOD. C: Mpv17 expressing (NIX15) cells treated as in A. D: Reference spectrum of the DMPO-OH adduct generated by reaction of DMPO with H₂O₂ upon UV radiation. Representative results from three independent experiments.

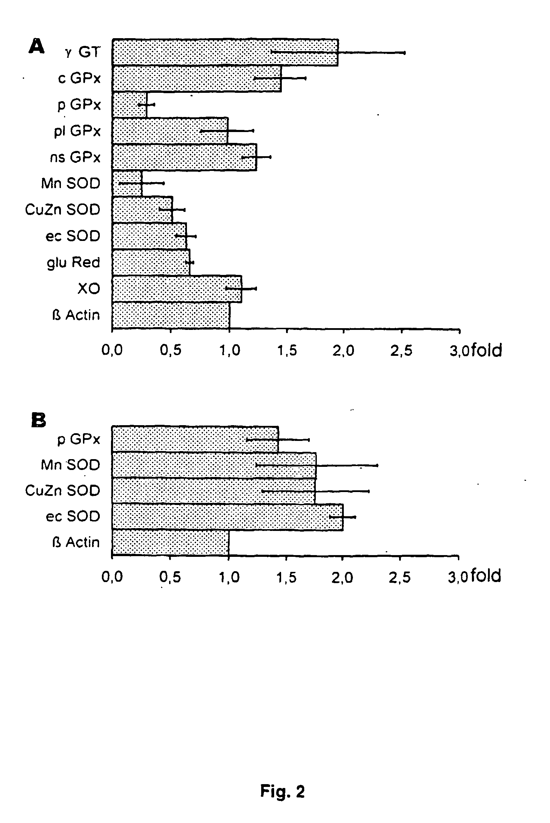

- **Figure 2**:: mRNA levels of fibroblasts in culture. A: Ratio of mRNA levels in Mpv17-/- versus Mpv17 expressing cells. B: Acivicin dependent ratio of mRNA levels in Mpv17-/- cells. β-actin mRNA was used for reference.
(Columns represent means ± S.D., n=3)

The following examples described below establish the usefulness of γ-GT inhibitors for the treatment of ROS induced chronic renal diseases and inner ear degenerative diseases and injuries.

### Example 1: ROS-generation in Mpv17 mice and its experimental investigation

The Mpv17 mouse strain is a genetically recessive rodent model of glomerulosclerosis (H. Weiher, T. Noda, D. A. Gray, A. H. Sharpe and R. Jaenisch (1990), Cell, 62, pp. 425-434) and sensineural deafness (A. M. Meyer zum Gottesberge, A. Reuter and H. Weiher (1996), European Archiv Otorhinolaryngology, 253, pp 470-474). Kidney and inner ear show marked changes in the extracellular matrix composition of glomerular and cochlear basement membrane components and of matrix metalloproteinase 2 (MMP-2) (A. Reuter, A. Nestl, R.M. Zwacka, J. Tuckermann, R. Waldherr, E. M. Wagner, M. Hoyhtya, A. M. Meyer zum Gottesberge, P. Angel and H. Weiher (1998), Molecular Biology of the Cell, 9, pp. 1675-1682). The Mpv17 gene, which is insertionally inactivated in mutant mice, codes for a peroxisomal hydrophobic 20 kDa protein with yet unknown molecular function (R. M. Zwacka, A. Reuter, E. Pfaff, J. Moll, K. Gorgas, M. Karasawa and H Weiher (1994), EMBO Journal 13, pp 5129-5134). A role of reactive oxygen species (ROS) in the pathogenesis was recently established, as oxygen radical scavengers attenuate the development and progression of the disease (C. J. Binder, H. Weiher, M. Exner and D. Kerjaschki (1999), American Journal of Pathology, 154, pp. 1067-1075).
In the experiments/examples described below ROS generated in Mpv17-/- cells were detected by electron spin resonance (ESR). Furthermore, activities and expression of enzymes of the antioxidant defense line were studied in Mpv17-/- kidneys and cells in culture. The results reveal an imbalance of the activity and/or gene expression of some protective enzymes. A novel route of γ-glutamyl transpeptidase (γ GT) in the development of oxidative damage is proposed in accordance with a pro-oxidant effect of up-regulated γ-glutamyl transpeptidase recently reported for short-term ischemia of rat kidney (Cutrin, Kidney International 57 (2000), 526-533). As in the Mpv17 animal model, γ-glutamyl-transpeptidase regulation may be important in the investigation of human kidney and inner ear disease.

Kidneys from background mice (CFW x Balb/c) and transgenic homozygous Mpv17-/- mice (H. Weiher, T. Noda, D. A. Gray, A. H. Sharpe and R. Jaenisch (1990), Cell, 62, pp. 425-434) were obtained at an age of 7 to 9 months and frozen in liquid nitrogen. LUSVX cells (SV40 immortalised fibroblasts from the lungs of Mpv17-/-mice), and NIX15 cells (Mpv+/+ cells derived from LUSVX cells by stable transfection of an expression construct for the human Mpv17 cDNA) (A. Reuter. A. Nestl, R. M. Zwacka, J. Tuckermann, R. Waldherr, E. M. Wagner, M. Hoyhtya, A. M. Meyer zum Gottesberge, P. Angel and H. Weiher (1998), Molecular Biology of the Cell, 9, pp. 1675-1682) were grown in DMEM including 10% fetal calf serum (FCS) and 1 µg/ml puromycin (NIX15 cultures) at 6.5 % CO₂ Cells were harvested by trypsinisation and each 5 x 10⁶ cells were washed in 1.5 ml of phosphate-buffered saline (PBS) before freezing the pellet in liquid nitrogen. Where indicated, Mn(III)tetrakis(4-benzoic acid)porphyrin (MnTBAP), final concentration 10 µM (Y. Noda, M. Kohno, A. Mori and L. Packer (1999), Methods in Enzymology, 299, pp. 28-34) (Calbiochem, Schwalbach, FRG), or the γ-GT inhibitor acivicin (AT-125), final concentration 50 µM (Sigma, Deisenhofen, FRG) were added in DMEM/10% FCS 18 h before harvesting the cells. Kidneys and cells were stored frozen at -80°C.

### Electron spin resonance (ESR) measurements were carried out as follows:

Radical production in lung fibroblasts from Mpv17-/- animals (LUSVX) was identified by ESR and compared to Mpv17 expressing fibroblasts (NIX15). For ESR determination of generated ROS (K. M. Faulkner, S. I. Liochev and I. Fridovich (1994), Journal Biological Chemistry, 269, pp. 23471-23476), 5,5 dimethyl-1-pyrroline N-oxide (DMPO) (Sigma, Deisenhofen, FRG) was used as spin trap. To 1 ml of cell suspension (1.5 x 10⁶/ml in PBS) a final concentration of 6.5 mM DMPO (kept under nitrogen at -20°C after charcoal filtration) was added. After 1 h at 37°C (for the samples represented in Fig.1 C below in the presence of 30 U/ml Superoxide dismutase) the samples were stored in liquid nitrogen. ESR was performed on a Bruker ESP300 instrument. The settings were: modulation amplitude: 2 G; gain: 1.6 x 10⁶, power: 40mW, sweep 100G/40s = 2.5 G/s. A reference spectrum was derived by reaction of DMPO with H₂O₂ upon UV radiation.

### Enzyme assays and glutathione assays were carried out as follows:

Cell fractions were prepared by differential centrifugation from 20% tissue and cell homogenates (w/v) in 10 mM potassium phosphate buffer, pH 7.0, containing 0.25 M sucrose, 150 mM potassium chloride, 1 mM EDTA, 2 mM dithiothreitol, and 50 µM phenylmethylsulfonyl fluoride. Enzyme activities were determined at 25°C: catalase in the 25,000 x g (10 min) pellet H. E. Aebi (1983), In Methods of Enzymatic Analysis (ed. H. U. Bergmeyer), Verlag Chemie, Weinheim, pp. 273-276); glutathione peroxidase (GPx) (R. A. Lawrence and R. F. Burk (1976), Biochemical Biophysical Research Communications, 71, pp. 952-958), GSSG reductase (Goldberg, D. M. S. R. F. (1983) In Methods of Enzymatic Analysis (ed. H. U. Bergmeyer) Verlag Chemie, Weinheim, pp. 258-265), glutathione transferase (GST) with 1-chloro-2,4-dinitrobenzene (CDNB) W. H. Habig, M. J. Pabst and W. B. Jakoby (1974), Journal Biological Chemistry, 249, pp. 7130-7139, and superoxide dismutase (SOD) (S. Marklund and G. Marklund (1974), European Journal of Biochemistry, 47, pp. 469-474) in the 105,000 x g (45 min) supernatant; γ-glutamyl transpeptidase (γ-GT)) (S. Marklund and G. Marklund (1974), European Journal of Biochemistry, 47, pp. 469-474) in the resuspended 105,000 x g pellet containing 1% Triton X-100. Protein was measured by the method of Bradford using bovine serum albumin as standard (M. M. Bradford (1976), Analytical Biochemistry, 72, pp. 248-254).

Glutathione equivalents (GSH + 2 GSSG) were assayed in kidney cytosols following the formation of 5-thio-2-nitrobenzoate (TNB) spectrophotometrically at 405 nm (T. P. Akerboom and H. Sies (1981), Methods in Enzymology, 77, pp. 373-382)

mRNA levels were also measured. In particular, levels of mRNA were determined by quantitative RT-PCR. Five µg of total RNA (F. M. Ausubel (1995) Short protocols in molecular biology: a compendium of methods from current protocols in molecular biology. Wiley Press, New York.) were used in each cDNA synthesis reaction (Superscript II reverse transcriptase system, Gibco). Primers were selected from published sequences: mouse γ-GT (Z. Z. Shi, G. M. Habib, R. M. Lebovitz and M. W. Lieberman (1995), Gene, 167, pp. 233-237), mouse cellular glutathione peroxidase (cGPx) (I. Chambers, J. Frampton, P. Goldfarb, N. Affara, W. McBain and P. R. Harrison (1986), EMBO Journal, 5, pp. 1221-1227), mouse plasma glutathione peroxidase (pGPx) (R. L. Maser, B. S. Magenheimer and J. P. Calvet (1994), Journal of Biological Chemistry, 269, pp. 27066-27073), mouse non-selenium glutathione peroxidase (nsGPx) B. Munz, S. Frank, G. Hubner, E. Olsen and S. Werner (1997), Biochemical Journal, 326, pp. 579-585), mouse phospholipid hydroperoxide glutathione peroxidase (PHGPx) (S. Nam, N. Nakamuta, M. Kurohmaru and Y. Hayashi (1997), Gene, 198, pp. 245-249), mouse glutathione reductase (glu red) (M. Tutic, X. A. Lu, R. H. Schirmer and D. Werner (1990), European Journal of Biochemistry, 188, pp. 523-528), mouse copper-zinc superoxide dismutase (CuZnSOD) (G. C. Bewley (1988), Nucleic Acids Research, 16, p. 2728), mouse manganese superoxide dismutase (MnSOD) (R. A. Hallewell, G. T. Mullenbach, M. M. Stempien and G. I. Bell (1986), Nucleic Acids Research, 14, p. 9539), mouse extracellular superoxide dismutase (ecSOD) (J. G. Suh, S. Takai, T. Yamanishi, T. Kikuchi, R. J. Folz, K. Tanaka, Y. S. Oh and K. Wada (1997), Molecules and Cells, 7, pp. 204-207) and mouse xanthine oxidase (XO) (M. Terao, G. Cazzaniga. P. Ghezzi, M. Bianchi, F. Falciani, P. Perani and E. Garattini (1992), Biochemical Journal, 283, pp. 863-870). Reactions were standardized using rat β-actin as a control. (U. Nudel, R. Zakut, M. Shani, S. Neumann, Z. Levy and D. Yaffe (1983) Nucleic Acids Research, 11, pp. 1759-1771). For each primer pair the cycle number was established in which the accumulation of reaction products was linear.

| gene (°C) | primer sequences 5'-3' | no. of cycles | annealing temp. |
|---|---|---|---|
| γ-GT | GCTGTCCCTGGTGAAATCCG | | |
| | GCATAGGCAAACCGAAAGGC | 36-40 | 56 |
| cGPx | GGGGCAAGGTGCTGCTCATT | | |
| | GTACGAAAGCGGCGGCTGTA | 26-30 | 59 |
| pGPx | CGAGTATGGAGCCCTCACCA | | |
| | CCAGCGGATGTCATGGATCT | 34-40 | 58 |
| nsGPx | GCTTCCACGATTTCCTGGGA | | |
| | TGTTTGGCTTCCTCTTCGGA | 26-30 | 56 |
| pIGPx | TCTGGCAGGCACCATGTGTG | | |
| | ATCACCTGGGGCTCCTCCAT | 22-26 | 59 |
| glu red | AATTCAGTTGGCATGTCATCAAGCA | | |
| | CTGTGTGAACTTCAACACCTCCACG | 28-34 | 59 |
| CuZnSOD | TGGCGATGAAAGCGGTGTGC | | |
| | GCGGCTCCCAGCATTTCCAG | 22-24 | 59 |
| MnSOD | AACAACCTCAACGCCACCGA | | |
| | CAATCCCCAGCAGCGGAATA | 28-30 | 59 |
| ecSOD | CGGCCTGTGGCTCTGTCACCATGT | | |
| | CACCACGAAGTTGCCAAAGTCGCC | 28-32 | 59 |
| XO | CCTGCTTGACCCCCATCTGC | | |
| | CGGACTTGACCTGCTTGCCA | 30-32 | 58 |
| β-actin | TCATAGATGGGCACAGTGTG | | |
| | CTAAGGCCAACCGTGAAAAG | 22-26 | 59 |

Reactions were performed for 30 s at the annealing temperature, 40 s at 72°C for synthesis and 30 s at 95°C for denaturation. Amplified mRNA was separated on 1.5 % agarose gels in Tris borate buffer (F. M. Ausubel (1995) Short protocols in molecular biology: a compendium of methods from current protocols in molecular biology. Wiley Press, New York) visualized with vista green (Amersham, Freiburg, FRG) and quantified with a FLA2000 Imager (FUJI).

### Example 2: Mpv17 expression diminishes superoxide production in fibroblasts from Mpv17 -/- mice

Spin-adducts formed in the presence of Mpv17-/- cells (LUSVX) showed a profile as depicted in Figure 1A, thus characteristic of a profile of DMPO-OH adducts (compare to Figure 1 D). Adducts were found mostly in the supernatant rather than within the cells (not shown) indicating that they were either formed within the cell and secreted, or generated by ROS released into the medium. When the reaction was performed in the presence of exogenous superoxide dismutase (SOD) the DMPO-OH peak decreased to about one-third (Figure 1 B) indicating that most of the stable DMPO-OH adducts were generated indirectly via an *OOH-adduct formed by the reaction with superoxide. With Mpv17 expressing NIX 15 cells, generated by transfection of Mpv17-/-cells (LUSVX), the concentration of DMPO-OH was decreased by about 35% as compared to Mpv17-/- cells (LUSVX) (Figure 1C).

### Example 3: Changes in the glutathione cycle in kidneys of Mpv17-/- mice

Since cultured LUSVX cells (Mpv17-/-) produce elevated levels of superoxide and oxidative damage has been shown to be causal to the development of the disease in Mpv17-/- mice (C. J. Binder, H. Weiher, M. Exner and D. Kerjaschki (1999), American Journal of Pathology, 154, pp. 1067-1075), enzymatic activities relevant to superoxide production were determined in kidneys of Mpv17-/- mice showing progressed glomerulosclerosis at an age of 7-9 months (C. J. Binder, H. Weiher, M. Exner and D. Kerjaschki (1999), American Journal of Pathology, 154, pp. 1067-1075) (Table 1). As compared to the corresponding Mpv17+/+ kidneys, no significant difference in glutathione levels and in the activities of superoxide dismutase (SOD), catalase, GSSG reductase and glutathione transferase (GST) was observed in the kidneys of both mouse strains. However, in kidneys of Mpv17-/- mice γ-glutamyl transpeptidase (γ-GT) activity was increased by about two-fold, whereas glutathione peroxidase (GPx) activity was decreased by about one-third as compared to Mpv17+/+ animals. The following table illustrates the results:

**TABLE 1 Enzyme activities in kidneys of Mpv17 -/- and Mpv17+/+ 7-9 months old mice**

| | Mpv17 -/- | | Mpv17 +/+ |
|---|---|---|---|
| | mmol/g of kidney wet weight | | |
| GSH | 2.19 ± 0.28 | (93) ^{a)} | 2.35 ± 0.35 |
| | U / mg of protein | | |
| Superoxide Dismutase | 17.1 ± 1.6 | (102) | 16.7 ± 0.8 |
| Catalase | 381 ± 25 | (88) | 431 ± 16 |
| | nmol/min per mg of protein | | |
| Glutathione Peroxidase | 176 ± 12 | (68) | 259 ± 16 |
| GSSG Reductase | 98 ± 10 | (85) | 115 ± 10 |
| Glutathione Transferase | 508 ± 52 | (98) | 520 ± 36 |
| γ-Glutamyl Transpeptidase | 2,330 ± 295 | (197) | 1,180 ± 70 |

| | | | |
|---|---|---|---|
| Data are given as means ± SEM (n= 10 animals) ^{a)} % of Mpv17+/+ | | | |

### Example 4: Mpv17 dependent activities of γ-glutamyl transpeptidase and glutathione peroxidase in fibroblasts

Changes of enzyme activities determined in kidneys of Mpv17-/- mice were similarly observed when in cultured Mpv17-/- (LUSVX) cells were compared to Mpv17 expressing (NIX15) cells (Table 2). Most prominently, in Mpv17-/- cells the activities of γ-GT were elevated by about six-fold, whereas the activities of GPx were lowered by one-third. The similarity in the change of γ-GT and GPx activity measured in Mpv17+/+ and Mpv17-/- kidney and fibroblast culture suggests that these alterations occur at the cellular rather than the organismal level. The following table summarizes the results:

**TABLE 2 Enzyme activities in LUSVX and NIX15 fibroblasts**

| | LUSVX | | NIX15 |
|---|---|---|---|
| | (Mpv17 negative) | | (Mpv17 expressing) |
| | U/ mg of protein | | |
| Superoxide Dismutase (n=2) | 10.7 | (61)^{a)} | 17.6 |
| Catalase | 410 ± 60 | (87) | 470 ± 90 |
| | nmol/min per mg of protein | | |
| Glutathione Peroxidase | 53 ± 5 | (66) | 80 ± 7 |
| γ-Glutamyl Transpeptidase | 15 ± 0.4 | (600) | 2.5 ± 0.01 |

| | | | |
|---|---|---|---|
| Data are given as means ± SEM (n=3-4) ^{a)} % of NIX15 | | | |

### Example 5: Mpv17 dependent changes in mRNA expression in fibroblasts

mRNA levels of the γ-GT and GPx genes were examined by quantitative RT-PCR in Mpv17expressing (NIX15) and Mpv17-/- (LUSVX) cells respectively (see Figure 2A below). γ-GT specific mRNA was enhanced by about 2-fold in Mpv17-/- cells. The expression of cellular GPx (cGPx), plasma GPx (pGPx), phospholipid hydroperoxide GPx (PHGPx) and of the nonselenium dependent GPx (nsGPx) was investigated. In Mpv17-/- cells only pGPx expression was decreased by about 80% (Figure 2A), which is basically consistent with the alteration in the activity of GPx (see Tables 1 and 2 above). Predominantly, pGPx appears to account for the overall low GPx activity. Remarkably, the expression of PHGPx, an enzyme responsible for protection from phospholipid peroxidation (R. L. Maser, B. S. Magenheimer and J. P. Calvet (1994), Journal of Biological Chemistry, 269, pp. 27066-27073), was unaffected on the mRNA level.
The three different mouse SOD genes (CuZnSOD, MnSOD, ecSOD) show lower expression in Mpv17-/- cells, consistent with the lower SOD activity measured (see Table 2 above). No significant difference of xanthine dehydrogenase/ xanthine oxidase (XO) was detected on the mRNA level between Mpv17expressing and Mpv17 nonexpressing cells (see Figure 2A).

### Example 6: Inhibition of γ-glutamyl transpeptidase activity restores glutathione peroxidase activity in Mpv17-/- cells

An inverse regulation of the glutathione-utilizing enzyme activities γ-GT and GPx was observed in Mpv17-/- animals and cells (see Tables 1 and 2 above). Because Mpv17-/- cells produce increased superoxide, a presumed regulatory function of the superoxide anion was tested by growing Mpv17-/- cells (LUSVX) in the presence of the SOD mimic MnTBAP (Y. Noda, M. Kohno, A. Mori and L. Packer (1999), Journal Biological Chemistry, 269, pp. 23471-23476) Neither γ-GT nor GPx activities were changed significantly (Table 3) indicating that superoxide appears to have no role in the regulation of γ-GT and GPx in this system. Conversely, when Mpv17-/- cells were grown in the presence of acivicin, an efficient inhibitor of γ-GT, GPx activity was increased by about 1.6-fold. Thus, enzyme activities may be dependent on each other in a way that γ-GT downregulates the activity of GPx. This inverse effect was also detected at the level of stable mRNA, as γ-GT inhibition led to a significant increase of pGPx and SOD mRNA levels (see Figure 2B).

**TABLE 3 Activity of glutathione peroxidase and γ-glutamyl transpeptidase**

| in Mpv17 -/- cells (LUSVX) in presence of MnTBAP or acivicin | | | | |
|---|---|---|---|---|
| | LUSVX | LUSVX + MnTBAP | LUSVX | LUSVX + acivicin |
| | nmol/min per mg of protein | | | |
| Glutathione Peroxidase | 25.0 ± 1.0 | 26.1 v 0.6 | 33.1 ± 1.3 | 53.5 ± 2.0 |
| | | (104%) ^{a)} | | (162%) |
| | | | | |
| γ-Glutamyl Transpeptidase | 0.99 ± 0.02 | 0.94 ± 0.03 | 0.96 ± 0.02 | not detectable |
| | | (95%) | | |

| | | | | |
|---|---|---|---|---|
| Values are given as means ± SEM (n=3) ^{a)} % of controls | | | | |

### Example 7: Regulation of γ-GT and pGPx expression

The examples as documented herein above illustrate the following:

### a) Reactive oxygen species in Mpv17 -/- cells

Using the ESR method superoxide was detected as the ROS species released from Mpv17 -/- fibroblasts. Production and secretion of superoxide are lower in Mpv17 expessing as compared to nonexpressing Mpv17-/- cells. These data are in line with the significance of ROS in the generation of glomerular injury (R. J. Johnson, D. Lovett, R. I. Lehrer, W. G. Couser and S. J. Klebanoff (1994), Kidney International, 45, pp. 352-359) and with an analysis of Mpv17-/- kidneys and isolated glomeruli, in which antioxidants were successfully used for therapeutic intervention in Mpv17-/animals (C. J. Binder, H. Weiher, M. Exner and D. Kerjaschki (1999), American Journal of Pathology, 154, pp. 1067-1075).

### b) Activity and expression of oxidative enzymes

Activity and expression of enzymes involved in ROS and glutathione metabolism were determined in Mpv17-/- mice kidneys and fibroblasts in culture. An increase in γ-GT activity and a decrease in GPx activity were observed in both, Mpv17-/- kidneys and fibroblasts. In addition, a decrease in SOD activity was observed in Mpv17-/fibroblasts. At the mRNA level, a negative correlation between the expression of γ-GT and the expression of the GPx and the SOD genes was observed in Mpv17-/- cells. All the three different SOD mRNAs tested were significantly decreased, but only the plasma GPx gene expression was strongly diminished, the latter presumably accounting for the decrease of GPx activity measured.
Negative correlations between the activity and expression of γ-GT and of enzymes involved in GSH and ROS metabolism have been reported earlier. Thus, inverse changes of GPx and γ-GT activities under the condition of oxidative stress were determined in rats exposed to cigarette smoke (C. V. Anand, U. Anand and R. Agarwal (1996), Indian Journal Experimental Biology, 34, pp. 486-488) and in fetal mice exposed to alcohol (S. A. Amini, R. H. Dunstan, P. R. Dunkley and R. N. Murdoch (1996), Free Radical Biology and Medicine, 21, pp. 357-365). Similarly, an inverse relationship of γ-GT and CuZnSOD expression has been noted recently in rat livers after iron poisoning (N. Taniguchi and Y. Ikeda (1998), Advances in Enzymology and Related Areas of Molecular Biology, 72, pp. 239-278) But, in contrast to these previously described models, in the Mpv17 mouse model no chemical insult was applied.

### c) Regulation of γ-GT and pGPx expression

In the absence of Mpv17 protein the γ-GT gene is upregulated while the mRNA level of pGPx is decreased. The mouse γ-GT gene is a single copy gene underlying intricate contol mechanisms involving at least seven promoters (N. Taniguchi and Y. Ikeda (1998), Advances in Enzymology and Related Areas of Molecular Biology, 72, pp. 239-278). The membrane-bound γ-GT is involved in regulating cellular redox potential and intracellular GSH levels (T. C. Nichols, J. M. Guthridge, D. R. Karp, H. Molina, D. R. Fletcher and V. M. Holers (1998), European Journal of Immunology, 28, pp. 4123-4129). The activity of γ-GT can be increased by glutathione depletion (R. J. van Klaveren, P. H. Hoet, J. L. Pype, M. Demedts and B. Nemery (1997), Free Radical Biologoy and Medicine, 22, pp. 525-534) or by hyperoxia (A. Kugelman, H. A. Choy, R. Liu, M. M. Shi, E. Gozal and H. J. Forman (1994), American Journal Respiratory Cell and Molecular Biology, 11, pp. 586-592) in different systems.

pGPx is an extracellular peroxidase of the selenium-containing GPx family, using GSH as well as and thioredoxin and glutaredoxin as thiol substrates (M. Björnstedt, J. Xue, W. Huang, B. Akesson and A. Holmgren (1994), Journal of Biological Chemistry, 269, pp. 29382-29384). More abundant in kidney than in other tissues, pGPx is synthesized and secreted in the proximal tubules and in the glomeruli, consistent with its function in protecting kidney from extracellular oxidative damage (R. L. Maser, B. S. Magenheimer and J. P. Calvet (1994), Journal of Biological Chemistry, 326, pp. 579-585; D. M. Tham, J. C. Whitin, K. K. Kim, S. X. Zhu and H. J. Cohen (1998), American Journal of Physiology, 275, G1463-1471). Downregulation of pGPx as observed in Mpv17-/- cells weakens the protection against extracellular oxidative insult.

The γ-GT activity in Mpv17-/- cells controls the level of pGPx mRNA as γ-GT inhibition relieves this downregulation (Fig2b). This control might involve imbalanced levels of intra- or extracellular GSH or superoxide due to enhanced γ-GT activity, presumably mediated by the activation of superoxide responsive transcription factors such as NF-κB or AP-1 (H. L. Pahl and P. A. Baeuerle (1994), Bioessays, 16, pp. 497-502). However, superoxide removal does neither affect the γ-GT nor the GPx activity, arguing against superoxide as a regulator.

Several genes relevant to the development of the disease phenotype, i.e. MMP-2 and its regulator TIMP-2, have been shown to be upregulated in Mpv17-/- mice earlier (A. Reuter, A. Nestl, R. M. Zwacka, J. Tuckerman, R. Waldherr, E. M. Wagner, M. Hoyhtya. A. M. Meyer zum Gottesberge, P. Angel and H. Weiher (1998), Molecular Biology of the Cell, 9, pp. 1675-1682). Since antioxidant intervention is effective in phenotype prevention in our model (C. J. Binder, H. Weiher, M. Exner and D. Kerjaschki (1999), American Journal of Pathology, 154, pp. 1067-1075), these alterations should be consequences rather than causes to ROS generation. By contrast, the data presented here suggest that overproduction of γ-GT in these animals is causal to elevated ROS levels (see below).

### d) Origin of enhanced ROS levels in Mpv17 -/- mice

Enzymes most affected in Mpv17-/- kidneys and cells, γ-GT and pGPx, both exert their enzymatic activity predominantly in the extracellular space. In particular, γ-GT expression and activity are enhanced in the absence of the Mpv17 function. Cells overproducing γ-GT should be efficiently protected against intracellular oxidative injury by increased supply of intracellular GSH. Extracellular GSH is metabolised by γ-GT to glutamate and cysteinylglycine which in contrast to GSH can directly enter cells and thus provide them with a source of cysteine (M. W. Lieberman, A. L. Wiseman, Z. Z. Shi, B. Z. Carter, R. Barrios, C. N. Ou, P. Chevez-Barrios, Y. Wang, G. M. Habib, J. C. Goodman, S. L. Huang, R. M. Lebovitz and M. M. Matzuk (1996), Proceedings of the National Academy of Sciences of the U.S.A., 76, pp. 5606-5610). The latter is present at lowest concentration of all amino acids and a limiting component for intracellular de novo GSH synthesis. Thus, γ-GT, localized at the luminal surface of the renal proximal tubules, plays a key role in cysteine and glutathione homeostasis in maintaining cellular GSH levels (A. Kugelman, H. A. Choy, R. Liu, M. N. Shi, E. Gozal and H. J. Forman (1994), American Journal Respiratory Cell and Molecular Biology, 11, pp. 586-592).

At the same time, increased γ-GT activity might lead to a depletion of extracellular GSH and thereby weaken the resistance against extracellular ROS. In mice, however, this is unlikely, because plasma GSH levels are about 100-fold higher than in humans (O. W. Griffith and A. Meister (1979), Prodeedings of the National Academy of Sciences of the U.S.A., 76, pp. 5606-5610), that is well above a critical substrate concentration for pGPx activity of <0.5 µM (A. Wendel and P. Cikryt (1980), FEBS Letters, 120, pp. 209-211). Instead, increased γ-GT activity may directly enhance superoxide in the Mpv17-/- system. Such direct production of superoxide by γ-GT activity was recently demonstrated in an in vitro system containing GSH and transferrin as an iron source. It was shown that superoxide was generated by the reaction of the GSH breakdown product cysteinylglycine (R. Drozdz, C. Parmentier, H. Hachad, P. Leroy, G. Siest and M. Wellman (1998), Free Radicals Biology and Medicine, 25, pp. 786-792). Superoxide can instantly undergo a Fenton type reaction to turn into the highly noxic hydroxyl radical, causing lipid- and protein peroxidation (R. Drozdz, C. Parmentier, H. Hachad, P. Leroy, G. Siest and M. Wellman (1998), Free Radicals Biology and Medicine, 25, pp. 786-792). In vivo, hydroxyl radical generation and lipid peroxidation in the presence of metals and under conditions of enhanced γ-GT activity have been described earlier in rat liver (K. E. Brown, M. T. Kinter, T. D. Oberley and D. R. Spitz (1998), Free Radical Biology and Medicine, 24, pp. 545-555; A. A. Stark, E. Zeiger, D. A. Pagano (1993) Carcinogenesis, 14, pp. 183-189; A. Paolicchi, R. Tongiani, P. Tonarelli, M. Comporti and A. Pompella (1997), Free Radicals Biology and Medicine, 22, pp. 853-860).

The above described experiments clearly demonstrate that γ-GT activity plays a key role in the generation of extracellular ROS. Recently, γ-GT upregulation has been reported to be causal to oxidation damage during short-term ischemia of rat kidney and this effect was inhibitable by acivicin (J. C. Cutrin, B. Zingaro, S. Camandola, A. Boveris, A. Pompella and G. Poli (2000), Kidney International, 57, pp. 526-533). Thus, the use of γ-GT inhibitors provides a potent and useful treatment of ROS degenerated diseases and injuries in humans as well.

Mpv 17-/-mice, i.e. mice of the glomerulosclerosis reference strains are treated, in accordance with this invention, by oral administration of 5 to 50 mg/kg activicin (AT-125) for several weeks. The protective use of activicin is analyzed by pathological methods and/or molecular means.

## Claims

1. Use of γ-GT inhibitors for the preparation of a pharmaceutical composition for the treatment of a chronic degenerative disease, wherein said chronic degenerative disease is a chronic renal disease or a chronic inner ear degenerative condition or injury.

2. The use of claim 1 wherein said chronic renal disease is ROS induced.

3. The use of claim 2, wherein said chronic renal disease is selected from the group consisting of focal glomerulosclerosis, segmental glomerulosclerosis, minimal change nephrosis, inflammatory glomerulopathies, diabetic nephropathy and autoimmuno glomerulopathies.

4. The use of claim 1, wherein said chronic inner ear injury is ROS induced.

5. The use of claim 4, wherein said ROS induced inner ear injury is sensineural deafness induced by age, physiological status or metabolic status.

6. The use of claim 1, wherein said chronic inner ear degenerative condition is otosclerosis.

7. The use of any one of claims 1 to 6, wherein said γ-GT inhibitor is selected from the group consisting of AT-125, Acivicin or its derivatives, γ-glutamyl amino acids and peptides of the general formula γ-Glu-XY, pepticles of the general formula (CysGlyX), peptidomimetic glutathion analogues, compounds or derivatives of the type L-2-amino-4-boronobutanoic acid (ABBA), and anilides, such as γ-glutamyl-7-amido-4-methylcoumarin (γ-Glu-AMC).

8. The use of claim 7, wherein X and Y stand for any naturally occurring aminoacid or a modified aminoacid.

## Patentansprüche

1. Verwendung von γ-GT-Inhibitoren für die Herstellung eines Arzneimittels für die Behandlung einer chronischen degenerativen Erkrankung, wobei die chronische degenerative Erkrankung eine chronische Nierenerkrankung oder ein(e) chronische(r) degenerative(r) Zustand oder Schädigung des Innenohrs ist.

2. Verwendung gemäß Anspruch 1, wobei die chronische Nierenerkrankung ROSinduziert ist.

3. Verwendung gemäß Anspruch 2, wobei die chronische Nierenerkrankung ausgewählt ist aus der Gruppe bestehend aus fokaler Glomerulosclerose, segmentaler Glomerulosclerose, Minimal-Change-Nephrose, entzündlichen Glomerulopathien, diabetischer Nephropathie und Autoimmun-Glomerulopathien.

4. Verwendung gemäß Anspruch 1, wobei die chronische Schädigung des Innenohrs ROSinduziert ist.

5. Verwendung gemäß Anspruch 4, wobei die ROS-induzierte Schädigung des Innenohrs sensorineurale Schwerhörigkeit ist, die durch das Alter, den physiologischen Zustand oder den metabolischen Zustand induziert wird.

6. Verwendung gemäß Anspruch 1, wobei der chronische degenerative Zustand des Innenohrs Otosclerose ist.

7. Verwendung gemäß einem der Ansprüche 1 bis 6, wobei der γ-GT-Inhibitor ausgewählt ist aus der Gruppe bestehend aus AT-125, Acivicin oder seinen Derivaten, γ-Glutamyl-Aminosäuren und Peptiden der allgemeinen Formel γ-Glu-XY, Peptikeln der allgemeinen Formel (CysGlyX), peptidmimetischen Glutathion-Analoga, Verbindungen oder Derivaten des Typs L-2-Amino-4-boronobuttersäure (ABBA) und Aniliden, wie γ-Glutamyl-7-amido-4-methylcumarin (γ-Glu-AMC).

8. Verwendung gemäß Anspruch 7, wobei X und Y für jedwede natürlicherweise vorkommende Aminosäure oder eine modifizierte Aminosäure stehen.

## Revendications

1. Utilisation d'inhibiteurs de γ-GT pour la préparation d'une composition pharmaceutique destinée au traitement d'une maladie dégénérative chronique, dans laquelle ladite maladie dégénérative chronique est une néphropathie chronique ou un état ou une lésion dégénérative chronique de l'oreille interne.

2. Utilisation selon la revendication 1, dans laquelle ladite néphropathie chronique est induite par ROS.

3. Utilisation selon la revendication 2, dans laquelle ladite néphropathie chronique est choisie dans le groupe constitué par la glomérulosclérose focale, la glomérulosclérose segmentaire, le syndrome néphrotique à lésions glomérulaires minimes, les glomérulopathies inflammatoires, la néphropathie diabétique et les glomérulopathies auto-immunes.

4. Utilisation selon la revendication 1, dans laquelle ladite lésion chronique de l'oreille interne est induite par ROS.

5. Utilisation selon la revendication 4, dans laquelle ladite lésion de l'oreille interne induite par ROS est une surdité sensorineurale induite par l'âge, un état physiologique ou un état métabolique.

6. Utilisation selon la revendication 1, dans laquelle ledit état dégénératif chronique de l'oreille interne est l'otosclérose.

7. Utilisation selon l'une quelconque des revendications 1 à 6, dans laquelle ledit inhibiteur de γ-GT est choisi dans le groupe constitué par l'AT-125, l'acivicine ou ses dérivés, les acides aminés γ-glutamyl et les peptides de formule générale γ-Glu-XY, les peptides de formule générale (CysGlyX), les analogues peptidomimétiques du glutathion, les composés ou dérivés du type acide L-2-amino-4-boronobutanoïque (ABBA), et les anilides tels que la γ-glutamyl-7-amido-4-méthylcoumarine (γ-Glu-AMC).

8. Utilisation selon la revendication 7, dans laquelle X et Y représentent n'importe quel acide aminé naturel ou un acide aminé modifié.
